# EUROPEAN PATENT SPECIFICATION

(11) **EP 2 066 258 B1**
(45) Date of publication and mention of the grant of the patent: **30.11.2016**
(21) Application number: 07839089.5
(22) Date of filing: 28.09.2007
(51) Int. Cl.: A61C 13/00, A61K 6/083

(54) **METHODS OF MANUFACTURING DENTAL APPLIANCES USING SURFACE TREATING COMPOSITIONS**
VERFAHREN ZUR HERSTELLUNG ZAHNÄRZTLICHER ANWENDUNGEN ÜBER OBERFLÄCHENBEHANDLUNGS-ZUSAMMENSETZUNGEN
PROCÉDÉS DE FABRICATION D'APPAREILS DENTAIRES UTILISANT DES COMPOSITIONS DE TRAITEMENT DE SURFACE

(30) Priority: 28.09.2006 US 528781
(43) Date of publication of application: 10.06.2009
(73) Proprietor: DENTSPLY International Inc., York, PA 17405-0872 (US)
(72) Inventor: SUN, Benjamin Jiemin, York PA 17402 (US); YOUNG, Andrew Mathias, Dallastown PA 17313 (US)
(74) Representative: Hartz, Nikolai
(86) International application number: PCT/US2007/021063
(87) International publication number: WO 2008/039544

(56) References cited:
- US-A- 6 077 075
- US-B2- 6 881 360

## Description

### BACKGROUND OF THE INVENTION

### Field of the Invention

The present invention relates generally to methods for making dental appliances and prostheses, particularly dentures. Compositions containing polymerizable acrylic compounds, a polymerization system, and solvent are used in treating the surface of a denture baseplate. This treatment results in effectively removing wax residue from the baseplate and improving its bonding strength to subsequently applied materials.

### Brief Description of the Related Art

Dental appliances include many devices such as, for example, full dentures, removable partial dentures, relines of full and partial dentures, nightguards, occlusal splints, and the like. Many conventional dentures are made from an acrylic base having a set of artificial teeth embedded therein.

In one method for making a denture, a processed baseplate is first prepared. Following this technique, a dentist first takes an impression of a patient's dental anatomy. A paste-like material, such as an alginate, is placed in a standard or custom-made impression tray. The dentist inserts the tray in the mouth of a patient and he or she bites down into the tray. Separate impression trays for the upper and lower dental arches are used. The dentist removes the trays from the patient's mouth and sets them aside to harden. The hardened impressions are then sent to a dental laboratory. There, a dental technician prepares casts (models) of the upper and lower arches by pouring dental plaster into the hardened impressions. The resulting plaster models have shaped surfaces closely representing the patient's upper and lower arches. A thin, smooth coating of a releasing agent, for example, Alcote® (available from Dentsply International), is applied to each dental model and the models are placed in a conditioning oven.

The respective upper and lower denture baseplates are prepared by molding a polymerizable material over the warm models. The polymerizable resin is applied so that it completely covers the model surfaces. A thin layer of an air barrier coating may be applied over the surfaces of the resin-coated models to ensure optimum curing. Each resin-coated model is placed in a light-curing unit and irradiated with light so that the resin hardens. After the light-curing cycle has been completed, each model, which supports a hardened baseplate, is removed from the unit and allowed to cool. Then, the hardened baseplates are removed from the respective models. The dental laboratory technician next mounts wax occlusal rims over the baseplates. The wax rim baseplates are returned to the dentist so they can be evaluated for fit in the patient's mouth. Then, the completed occlusal registration is articulated and the wax rims are removed.

In one method, a wax denture is built on the processed baseplate. Referring to a method for making an upper denture, wax is added to the processed baseplate and a set of artificial teeth are positioned in the wax.

Following this conventional method, a dental flask having two portions is chosen and prepared. The processed baseplate with completed tooth arrangement is placed in the flask containing a suitable investing medium. Then, the flask is heated to eliminate the wax. Upon melting, the wax flows out of the flask. Normally, a substantial portion of the wax is melted and removed from the flask using this step. Removing wax from inside of the flask leaves an interior cavity having a shape of a denture. It is important that the baseplate be substantially clean of any wax residue. Usually, the processed baseplate's surface must be ground to remove the residual wax. This treatment allows for bonding of the baseplate to subsequently applied acrylic resins as discussed further below. However, grinding of the baseplate can be difficult, since the baseplate is locked in invested dental stone in the flask. Thus, the baseplate is often rinsed with boiling water in an effort to substantially remove the wax residue from the baseplate's surface. In a next step, a polymerizable acrylic composition is "packed into" into the interior cavity of the flask. The acrylic composition is heated so that it bonds to the teeth and baseplate. When this acrylic composition cures and hardens, it will hold the artificial teeth in position. In other words, the acrylic composition replaces the temporary wax layer and is incorporated into the final denture.

One disadvantage with making dentures using prior art methods relates to the bonding of the acrylic composition to the baseplate. The surface of the cured acrylic baseplate needs to be free of wax and other contaminants. When there is a clean interface between the cured baseplate surface and acrylic layer, a relatively strong and durable bond can form between the baseplate and acrylic. Rinsing the baseplate with boiling water, as is done in conventional methods, can be somewhat effective for removing softer baseplate wax. However, even if the rinsing step is repeated several times, some residual wax is likely to remain on the surface of the baseplate.

One prior art method used for making dentures is described in Bedard et al., U.S. Patent 6,077,075, on which the two-part form of claim 1 is based. Advantageous embodiments are disclosed in the dependent claims 2-12

. In this method, a laminate blank with a soft layer and hard, strengthening layer is provided. The blank is heated so that it is moldable, and then molded into contact with a study cast. The molded laminate is trimmed to form a "try-in" baseplate. A temporary wax plate, bearing artificial teeth, is formed on the try-in baseplate. After testing the baseplate in a patient's mouth and making any necessary adjustments, the baseplate is placed in a dental flask. In the flasking process, the wax layer is heated so that it flows out. A polymerizable overlayer is substituted in place of the wax layer. The '075 Patent describes introducing a detergent or wash formulation into the interior space of the flask to assure complete wax removal.

In another method, Stange et al., U.S. Patent 6,881,360 discloses a process for producing a dental prosthesis such as a denture using an insulated film. A working model of a patient's dental anatomy is first constructed and a baseplate is applied to the working model. Then, a polymeric composition comprising a multifunctional urethane methacrylate / acrylate resin is applied to the baseplate and subsequently hardened. An insulating film is applied to the baseplate in order to obtain a tooth impression in wax. A dental investment material is used to form the framework for the teeth. The wax material is then boiled out by adding hot boiling water to the investment material, and the insulating film is removed. The hollow space created by removing the wax is filled with a polymeric material, which is subsequently hardened to form the final denture.

Boiling out the wax from inside of a dental flask, as described in the foregoing patents, is one method used to remove wax from a processed baseplate. However, even when boiling water is introduced into the flask repeatedly, some residual wax is likely to remain on the baseplate. There is a need for a composition that can effectively remove wax residue and treat the surface of baseplate as a step in manufacturing the denture or other dental appliance. The present invention provides such a dental composition. The invention also provides methods for making dental appliances using such wax-removing and surface-treating compositions. These and other objects, features, and advantages of the invention are evident from the following description and illustrated embodiments.

### SUMMARY OF THE INVENTION

The present invention relates to methods for making dental appliances and prosthesis, particularly dentures. In one embodiment, a baseplate having an outer surface is provided. A temporary wax layer is molded over the baseplate surface and a set of artificial teeth are pressed in the wax layer so as to form a try-in baseplate. A dentist places the baseplate in a patient's mouth to evaluate the base for fit. After the baseplate has been removed, it is treated with heat to substantially remove the wax layer. Then, a first polymerizable acrylic dental composition is applied to the baseplate so that the composition can at least partially diffuse therein. A second acrylic composition is subsequently applied to the baseplate. Ultimately, the first and second polymerizable resins, baseplate, and set of artificial teeth are bonded together, and the acrylic resins are cured to form a dental appliance.

The first acrylic composition comprises a polymerizable acrylic compound; a polymerization initiation system, capable of being activated by light or heat, for polymerizing the composition; and a solvent capable of at least partially solubilizing wax material. Preferably, a mixture of heptane and methyl acetate solvents is used. The compositions are able to remove wax residue effectively and improve the bonding strength of a denture baseplate.

In a second embodiment, the method involves providing a baseplate with mounted wax occlusal rims. A dentist places the baseplate in a patient's mouth to evaluate the base for fit. The wax rims are subsequently removed and discarded. Then, a first polymerizable acrylic dental composition is applied to the surface of the baseplate so that the composition can remove any remaining wax material from the baseplate. A second acrylic composition is later applied to the baseplate as an overlayer, and a set of artificial teeth are pressed into the overlayer. Upon cooling, the overlayer forms a stable, uncured layer. Then, the first and second acrylic compositions are polymerized and hardened to form a cured dental appliance.

### BRIEF DESCRIPTION OF THE DRAWINGS

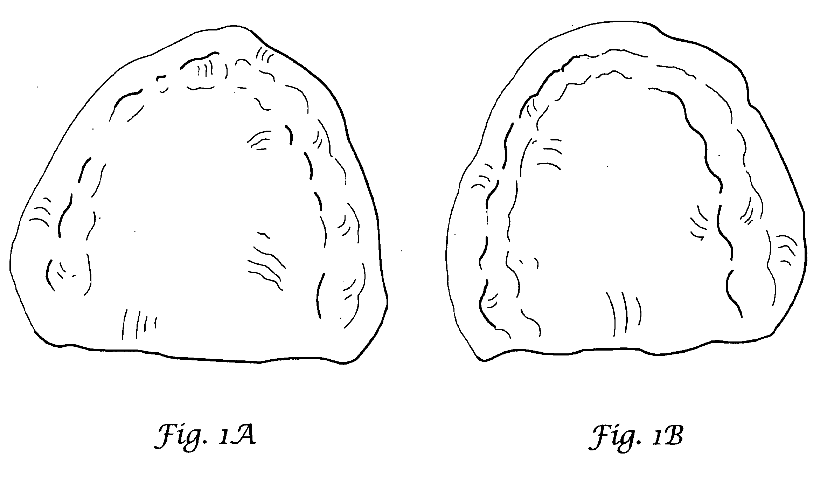

FIG. 1A is a photographic image of a denture baseplate treated with a wax-removing/ surface-treating composition of the present invention.
FIG. 1B is a photographic image of a comparative denture baseplate that has not been treated with a wax-removing/ surface-treating composition of the invention.

### DETAILED DESCRIPTION OF THE PREFERRED EMBODIMENTS

The present invention relates to methods of using a polymerizable dental composition to remove wax residue and treat the surface of a baseplate during the fabrication of dentures and other dental appliances.

The polymerizable dental composition used in accordance with this invention comprises a polymerizable acrylic compound; a polymerization initiation system, capable of being activated by light or heat, for polymerizing the composition; and a solvent capable of at least partially diffusing into the surface of the baseplate. The composition can be prepared using the following components.

### Polymerizable Acrylic Compounds

Polymerizable acrylic compounds that can be used in the composition of this invention, include, but are not limited to, mono-, di- or poly-acrylates and methacrylates such as methyl acrylate, methyl methacrylate, ethyl acrylate, isopropyl methacrylate, n-hexyl acrylate, stearyl acrylate, allyl acrylate, glycerol diacrylate, glycerol triacrylate, ethyleneglycol diacrylate, diethyleneglycol diacrylate, triethyleneglycol dimethacrylate, tetraethylene glycol di(meth)acrylate, 1,3-propanediol diacrylate, 1,3-propanediol dimethacrylate, trimethylolpropane tri(meth)acrylate, 1,2,4-butanetriol trimethacrylate, 1,4-cyclohexanediol diacrylate, 1,4-cyclohexanediol dimethacrylate, 1,6-hexanediol di(meth)acrylate, pentaerythritol triacrylate, pentaerythritol tetraacrylate, pentaerythritol tetramethacrylate, sorbitol hexacrylate, 2,2-bis[4-(2-hydroxy-3-acryloyloxypropoxy)phenyl]propane; 2,2-bis[4-(2-hydroxy-3-methacryloyloxypropoxy)phenyl]propane (Bis-GMA); 2,2-bis[4-(acryloyloxy-ethoxy)phenyl]propane; 2,2-bis[4-(methacryloyloxy-ethoxy)phenyl]propane (or ethoxylated bisphenol A-dimethacrylate) (EBPADMA); urethane di(meth)acrylate (UDMA), diurethane dimethacrylate (DUDMA), 4,13-dioxo-3,14 dioxa-5,12-diazahexadecane-1,16-diol diacrylate; 4,13-dioxo-3,14 dioxa-5,12-diazahexadecane-1,16-diol dimethacrylate; the reaction product of trimethyl 1,6-diisocyanatohexane and bisphenol A propoxylate and 2-hydroxyethyl methacrylate (TBDMA); the reaction product of 1,6 diisocyanatohexane and 2-hydroxyethyl methacrylate modified with water (HDIDMA); the reaction product of 1,6 diisocyanatohexane and 2-hydroxyethyl acrylate modified with water (HDIDA); polyurethane dimethacrylate (PUDMA); alkoxylated pentaerythritol tetraacrylate; polycarbonate dimethacrylate (PCDMA); the bis-acrylates and bis-methacrylates of polyethylene glycols; and copolymerizable mixtures of acrylated monomers and acrylated oligomers.

In addition to the foregoing polymerizable acrylic compounds, the composition may contain acidic monomers such as dipentaerythritol pentacrylate phosphoric acid ester (PENTA); bis[2-(methacryloxyloxy)-ethyl]phosphate; and vinyl compounds such as styrene, diallyl phthalate, divinyl succinate, divinyl adipate and divinylphthalate. Diluent polymerizable monomers also may be added to the composition. For example, hydroxy alkyl methacrylates, ethylene glycol methacrylates, and diol methacrylates such as tri(ethylene glycol) dimethacrylate (TEGDMA) may be added to reduce viscosity and make the composition more suitable for application. A polymerizable acrylic compound can be used alone in the composition or mixtures of the compounds can be used.

### Solvents

The composition of this invention further contains a solvent capable of at least partially diffusing into the surface of the baseplate. The solvent is also capable of at least partially solubilizing wax material. Examples of suitable solvents include, but are not limited to, acetone, pentane, hexane, octane, methyl pentane, dimethyl pentane, trimethyl pentane, methyl hexane, dimethyl hexane, methyl butane, dimethyl butane, trimethyl butane, cyclohexane, cycloheptane, mineral spirits, ethyl acetate, propyl acetate, ethers, methylene chloride, chloroform, cyclohexanone, heptane, methyl acetate, methyl ethyl ketone, methyl propyl ketone, and tetrahydrofuran, and the like. Preferably, a mixture of heptane and methyl acetate solvents is used. The heptane acts a "degreaser" to dissolve wax left on the base plate. The methyl acetate is able to penetrate into the cured baseplate material as described further below.

### Fillers

Conventional filler materials such as inorganic fillers, which can be naturally-occurring or synthetic, can be added to the composition. Such materials include, but are not limited to, silica, titanium dioxide, iron oxides, silicon nitrides, glasses such as calcium, lead, lithium, cerium, tin, zirconium, strontium, barium, and aluminum-based glasses, borosilicate glasses, strontium borosilicate, barium silicate, lithium silicate, lithium alumina silicate, kaolin, quartz, and talc. Preferably, the silica is in the form of silanized fumed silica. Preferred glass fillers are silanized barium boron aluminosilicate and silanized fluoride barium boron aluminosilicate. Organic particles such as poly(methyl methacrylate), poly(methyl/ethyl methacrylate), crosslinked polyacrylates, polyurethanes, polyethylene, polypropylene, polycarbonates and polyepoxides , and the like also can be used as fillers.

The inorganic filler particles can be surface-treated with a silane compound or other coupling agent to improve bonding between the particles and resin matrix. Suitable silane compounds include, but are not limited to, gamma-methacryloxypropyltrimethoxysilane, gamma-mercaptopropyltriethoxysilane, gamma-aminopropyltrimethoxysilane, and combinations thereof.

### Polymerization System

A polymerization system can be used in the composition of this invention, which initiates polymerization (hardening) of the composition by a light-curable or heat-curable reaction. In one embodiment, a photoactive agent such as, for example, benzophenone, benzoin and their derivatives, or alpha-diketones and their derivatives is added to the composition in order to make it light-curable. A preferred photopolymerization initiator is camphorquinone (CQ). Photopolymerization can be initiated by irradiating the composition with blue, visible light preferably having a wavelength in the range of about 400 to about 500 nm. A standard dental blue light-curing unit can be used to irradiate the composition. The camphorquinone (CQ) compounds have a light absorbency maximum of between about 400 to about 500 nm and generate free radicals for polymerization when irradiated with light having a wavelength in this range. Photoinitiators selected from the class of acylphosphine oxides can also be used. These compounds include, for example, monoacyl phosphine oxide derivatives, bisacyl phosphine oxide derivatives, and triacyl phosphine oxide derivatives. For example, 2,4,6-trimethylbenzoyl-diphenylphosphine oxide (TPO) can be used as the photopolymerization initiator. In one embodiment, a material referred to as "ALF" comprising camphorquinone (CQ); butylated hydroxytoluene (BHT); N,N-dimethylaminoneopentyl acrylate, and methacrylic acid can be used in the composition.

In another embodiment, heat-activated polymerization initiators, such as peroxides, can be added to make the composition heat-curable. The peroxides generate free radicals to initiate polymerization and hardening of the composition at elevated temperature. Peroxides such as dibenzoyl peroxide (BPO), di-p-chlorobenzoyl peroxide, di-2,4-dichlorobenzoyl peroxide, tertiary butyl peroxybenzoate, methyl ethyl ketone peroxide, ditertiary butyl peroxide, dicumyl peroxide and cumene hydroperoxide, and the like can be used.

In addition to the photoactive and heat activated agents, the composition may include a polymerization inhibitor such as, for example, butylated hydroxytoluene (BHT); hydroquinone; hydroquinone monomethyl ether; benzoquinone; chloranil; phenol; butyl hydroxyanaline (BHT); tertiary butyl hydroquinone (TBHQ); tocopherol (Vitamin E); and the like. Preferably, butylated hydroxytoluene (BHT) is used as the polymerization inhibitor. The polymerization inhibitors act as scavengers to trap free radicals in the composition and to extend the composition's shelf life.

The above-described composition can be used in accordance with the present invention to fabricate dental appliances and prosthesis such as, for example, full dentures, removable partial dentures, relines of full and partial dentures, nightguards, occlusal splints, and the like. The composition is particularly suitable for making dentures. The method for making the denture is described hereinafter primarily as a method for making an upper denture; however, it should be understood that the method can be used to make both upper and lower dentures as well as other dental appliances.

In one embodiment of the method of this invention, a denture base (baseplate) is first prepared using conventional acrylic resins and techniques. By the term, "acrylic resin" as used herein, it is meant a resinous material comprising at least one polymerizable acrylic compound as described above. The acrylic resin used to make the denture base may contain the same polymerizable acrylic compounds used to prepare the wax-removing/surface-treating composition as described above. Alternatively, the acrylic resin for the denture base may contain different polymerizable acrylic compounds.

In a second embodiment, a light-curable polymerizable material is used to form the denture baseplate. A preferred light-curable resin is available under the tradename, Eclipse®, from Dentsply International, Inc. (York, PA), and the baseplate may be prepared according to instructions from the manufacturer. Alternatively, a "wax-like" polymerizable material may be used to form the baseplate. By the term, "wax-like" as used herein, it is meant any material which is flowable (fluid) above 40°C and which becomes dimensionally stable (i.e., it solidifies and is non-fluid) at a temperature of at least and below 23°C. Flowable wax-like material having a temperature in the range of 40°C to 100°C becomes dimensionally stable within five minutes by cooling it to an ambient temperature in the range of 0°C to 37°C. Wax-like polymerizable materials include, for example, methacrylate (or acrylate) compounds prepared by reaction of a urethane pre-oligomer with hydroxylalkylmethacrylate. Such wax-like polymerizable materials are described in Sun et al., U.S. Patents 6,592,369; 6,799,969 and 7,024, 770. In still another method for making a baseplate, a thermoplastic material such as nylon or polycarbonate is molded and shaped to form a baseplate structure.

After the baseplate has been initially prepared, its outer surface is roughened-up with a coarse bur. This roughening-up step reduces the thickness of the baseplate and helps improve mechanical bonding between its surface and acrylic compositions that will be applied later. A dental laboratory technician places wax occlusal rims over the baseplate and sends it to a dentist for evaluation of fit and comfort in a patient's mouth. The dentist may adjust the wax rims for proper lip support and occlusion. Then, the baseplate is returned to the dental laboratory, where the wax rims are removed and discarded.

Next, the laboratory technician places a temporary wax set-up resin onto the roughened outer surface of the baseplate. The artificial teeth are pressed into the wax resin while the resin is in a softened condition. The wax resin is contoured so that it mimics natural dentition. The denture base, with temporary wax and embedded teeth, is sent back to the dentist so that he or she can place it in a patient's mouth as a "try in." Here, the dentist may wish to make adjustments to the "try-in" denture with wax layer and embedded teeth according to the dental anatomy of the patient. Then, the try-in denture is returned to the dental laboratory so that the final denture can be prepared.

The try-in denture is placed in a conventional dental flask filled with dental stone or gypsum. The dental flask has two portions, a bottom half-portion and upper half-portion. The flask portions are closed together and the flask is heated to melt the wax holding the artificial teeth. For example, the flask can be heated in a tank of boiling water. Upon melting, the wax flows out of the flask. This step may be referred to as "boiling out the wax" or the "lost wax" technique. Then, the flask container is opened and each flask portion is placed face-up on a bench or other suitable support surface. At this point, one portion of the flask contains the denture baseplate embedded in dental stone, and the other portion of the flask contains the set of artificial teeth embedded in dental stone. While the baseplate is seated in half-portion of the flask, it is rinsed several times with boiling water. A clean towel is used to wipe the baseplate dry.

After rinsing the baseplate with boiling water, the plate may still contain small amounts of wax residue that need to be removed. The composition of this invention, as .described above, is used to remove the wax material. This composition can be referred to as a first polymerizable acrylic composition and should be brushed onto the baseplate - it is preferably applied as an overlayer at least three to four times. In addition, the first polymerizable acrylic composition can be applied to the embedded artificial teeth so as to remove any wax residue from the tooth surfaces. Treating the baseplate and artificial teeth in this manner causes the acrylic composition to partially diffuse into the surfaces of the baseplate and teeth. This leads to the baseplate and teeth having enhanced bonding strength as described in further detail below.

Immediately after the final overlayer of acrylic composition has been applied, the upper half-portion of the flask (containing the set of artificial teeth) is closed over the bottom half-portion (containing the baseplate). The flask is still relatively warm at this point - it is typically at a temperature in the range of about 50° to about 55°C. The flask is kept closed for a sufficient period of time, preferably 10 to 15 minutes. Then, the flask is opened and the upper and bottom half-portions are allowed to cool.

After the flask has cooled completely, a second polymerizable acrylic composition is packed into the upper half-portion of the flask containing the set of artificial teeth. The second acrylic composition may contain different polymerizable acrylic compounds as used in the first acrylic composition. Alternatively, the acrylic resin for the denture base may contain the same polymerizable acrylic compounds. The second acrylic composition also contains a polymerization system, which may comprise light-curable and heat-activated initiators as described above. Alternatively, a self (chemically)-curable polymerization system can be used. Such self-curable systems are usually made of two parts. One part contains a free radical polymerization accelerator such as a tertiary amine and the other part contains a polymerization accelerator such as a peroxide. When the parts are mixed together, the amine and peroxide react with each other and initiate polymerization and hardening of the composition.

The second acrylic composition differs from the above-described first acrylic composition in that it does not contain any solvents for solubilizing the wax material. Also, at this point, a (methyl methacrylate) liquid monomer can be applied to wet the surface of the baseplate seated in the lower half-portion. Then, the flask is closed and heated for a sufficient period of time (typically a 2 hour short heat-cure or 9 hour long heat-cure). As the upper and lower half-portions of the flask are pressed together, the acrylic resin, baseplate, and set of artificial teeth are bonded together. The first and second polymerizable acrylic compositions polymerize and harden, thereby fully setting the artificial teeth in the denture baseplate. Eventually, the flask is opened and the final denture appliance with embedded artificial teeth is removed therefrom. Lastly, the denture appliance is finished and polished using conventional techniques.

As discussed above, in a preferred embodiment, the composition of this invention (first polymerizable acrylic composition) contains a mixture of the solvents, heptane and methyl acetate. The heptane solvent acts as a degreaser, dissolving residual wax located on the cured surface of the baseplate and artificial tooth surfaces. The methyl acetate solvent works on the baseplate and artificial tooth surfaces so that polymerizable acrylic compounds can penetrate into the baseplate material and artificial teeth. That is, the mixture of heptane and methyl acetate solvents attacks the baseplate material and artificial teeth, allowing the polymerizable acrylic compounds to at least partially diffuse therein. For example, the methacrylate resin in the composition can penetrate partially into the surface of the baseplate. The methacrylate resin will later polymerize to form interpenetrating polymer networks with the subsequently applied second polymerizable acrylic composition as described above. As the resins polymerize, a strong bonding interface is formed. The volatile solvent included in the first polymerizable acrylic composition will evaporate quickly upon heating of the baseplate or over a short period of time when the baseplate is kept at room temperature.

In another embodiment of the method of this invention, the dental laboratory prepares a processed baseplate. As described above, wax occlusal rims are placed over the baseplate, and the laboratory sends the baseplate to the dentist for evaluation of fit in a patient's mouth. The dentist may adjust the wax rims for proper lip support and make occlusal and aesthetic markings on the wax rims. Then, the baseplate is returned to the dental laboratory. The baseplate is mounted on a dental stone model (cast) replicating the patient's dental anatomy, and the occlusal and aesthetic markings from the baseplate are transferred to the model. The wax rims are then removed and discarded. While the baseplate is still mounted on the model, it is rinsed several times with boiling water to substantially remove the wax material. After the baseplate has been rinsed with the boiling water, its outer surface may be roughened-up using a bur or diamond. This helps improve bonding to subsequently applied materials. Although the baseplate is rinsed with boiling water, it may still contain some wax residue that needs to be removed. The wax-cleaning / surface- treating composition of this invention, as described above, is used to remove such wax residue and is allowed to penetrate into the surface of the baseplate. It is preferred to use heat during the wax-cleaning / surface- treating process to enhance the effectiveness of wax removal and surface penetration. The artificial teeth are now ready to be set on the baseplate.

First, a sheet or rope of a polymerizable material, also referred to as a set-up resin, is placed on the outer surface of the baseplate. The surface of the set-up resin is melted with an electric spatula or hot air gun just prior to tooth placement. The teeth are then pressed into the set-up resin while the resin is in a softened condition. Then, a molten polymerizable material, also referred to as molten contour resin, which has been prepared previously by placing the resin in a melting pot set at a high temperature is applied so that it flows around the embedded artificial teeth. The contour resin should flow between the embedded teeth smoothly and evenly, because, once the resin hardened, it will hold the teeth in position. As the contour resin flows around the embedded teeth, it covers the set-up resin and any exposed baseplate. A wax-like polymerizable material, as described above can be used as the set-up and contour resins. Such resins are available from Dentsply International under the tradename, Eclipse® Set-Up and Contour resins. The resins are allowed to cool so as to form a stable, uncured acrylic layer. The uncured denture is now ready to be sent to the dentist for placement in a patient's mouth as a "try in" denture. The dental laboratory sends the uncured denture to the dentist.

The denture should be fitted so that it conforms tightly to the contours of the patient's oral cavity and there is good occlusion. Since the denture is uncured and has an extended working time, the dentist can make some minor chairside adjustments. Then, the denture device is sent back to the dental laboratory so that the final denture device can be prepared.

At the laboratory, the denture is placed back on the model (a new model may be prepared if needed), and an air barrier coating is applied so that it covers all external resin surfaces and embedded teeth. The denture and supporting model are placed in a conditioning oven and heated. The denture/model is removed from the conditioning oven and a model releasing agent is applied to the flange areas of the denture. Next, the denture/model is placed in a light-curing / heat-generating apparatus. The denture/model is heated to a suitable temperature and light-cured. After the light-curing cycle has been completed, the denture/model is removed and allowed to cool until the denture reaches ambient temperature. Then, the denture is separated from the model. Finally, the denture is finished and polished using conventional techniques.

The wax-removing / surface-treating composition of this invention may contain semi-crystalline components. For example, polymerizable acrylic compounds such as 1,4-cyclohexanediol diacrylate, 1,4-cyclohexanediol dimethacrylate, 4,13-dioxo-3,14 dioxa-5,12-diazahexadecane-1,16-diol diacrylate; 4,13-dioxo-3,14 dioxa-5,12-diazahexadecane-1,16-diol dimethacrylate; the reaction product of 1,6 diisocyanatohexane and 2-hydroxyethyl methacrylate modified with water (HDIDMA); the reaction product of 1,6 diisocyanatohexane and 2-hydroxyethyl acrylate modified with water (HDIDA) can be semi-crystalline. Upon evaporation of the solvent from the composition, a solid layer of semi-crystalline acrylic material remains on the surface of the baseplate. The acrylic coating acts as an adhesion promoter and enhances bond strength with the second polymerizable acrylic composition applied subsequently to the cured baseplate surface. The dried composition of this invention forms a hard, non-sticky surface layer that provides an excellent interface for subsequently applied acrylics and other resins. Improved bond strength is obtained using the composition of this invention. In contrast, compositions that form a liquid or non-solid surface layer feel sticky or tacky. It is hard to handle and work with such sticky compositions. Moreover, at the uncured stage, the bond created with a liquid or non-solid surface layer between the baseplate and subsequently applied uncured solid acrylics, such as Eclipse® Contour and/or Set-up resins is not strong. As a result, the uncured solid acrylics can delaminate from the processed baseplate.

Components having high molecular weight and low tackiness at a temperature in the range of room temperature to 37°C are preferably included in the composition of this invention. For example, compounds such as the reaction product of trimethyl-1,6-diisocyanatohexane and bisphenol-A propoxylate and 2-hydroxymethylmethacryalte (TBDMA) have high molecular weight and low tackiness. These components provide rapid solidification of the polymerizable products upon solvent evaporation. The use of high molecular weight components results in low polymerization shrinkage and low stress polymerized products. The semi-crystalline components of this composition, as described above, are partially recrystallizable. Such rapid recrystallizable components provide rapid solidification of the polymerizable composition. The components have good flowability and dimensional stability depending upon the applied temperature and solvents used in the composition. When polymerized, the crystallized phase melts effectively resulting in volume expansion, which offsets polymerization shrinkage. Thus, the polymeric composition provides low shrinkage and low stress restoration.

The present invention is further illustrated by the following Examples, but these Examples should not be construed as limiting the scope of the invention.

### Examples

### Example 1

### Preparation of Oligomer

In the following Example 1, an oligomer that was to be used in the composition of this invention was prepared as follows. A reactor was charged with 1176 grams of trimethyl-1,6-diisocyanatohexane (5.59 mol) and 1064 grams of bisphenol A propoxylate (3.09 mol) under dry nitrogen flow and heated to about 65°C under positive nitrogen pressure. To this reaction mixture, 10 drops of the catalyst, dibutyltin dilaurate was added. The temperature of the reaction mixture was maintained between 65°C and 140°C for about 70 minutes and this step was followed by adding 10 drops of the catalyst, dibutyltin dilaurate. A viscous paste-like isocyanate end-capped intermediate product was formed, and the product was stirred for 100 minutes. To this intermediate product, 662 grams (5.09 mol) of 2-hydroxyethyl methacrylate and 7.0 grams of BHT as an inhibitor were added over a period of 70 minutes while the reaction temperature was maintained between 68°C and 90°C. After about five hours stirring under 70°C, the heat was turned off, and oligomer was collected from the reactor as semi-translucent flexible solid and stored in a dry atmosphere.

### Examples 2A - 2F

In the following Examples 2A-2F, different embodiments of the wax removal / surface treatment composition of this invention were prepared by mixing the components shown in Tables 1 and 2.

**Table 1. Polymerizable Dental Compositions of this Invention**

| **EXAMPLES** | **2A** | **2B** | **2C** |
|---|---|---|---|
| Heptane | 46.4 | | |
| Methyl Acetate | 48.3 | 70.2 | 87.0 |
| Compound of Example 1 | 3.6 | | 4.0 |
| Cyclohexane dimethanol dimethacrylate | | 14.0 | |
| 1,6-Hexanediol dimethacrylate | 1.62 | 10.0 | |
| Tetrahydrofurfuryl methacrylate | | 5.0 | |
| 2,4,6- Trimethylbenzoyldiphenylphosphine oxide (LUCIRIN TPO) | 0.02 | 0.2 | |
| Elvacite 2009 - Poly(MMA-co-EA) | | | 8.0 |
| Camphorquinone | 0.008 | 0.08 | 0.13 |
| N, N-dimethyl-aminoneopentyl acrylate | 0.027 | 0.27 | 0.47 |
| Methacrylic acid | 0.014 | 0.14 | 0.23 |
| Butylated hydroxytoluene | 0.001 | 0.01 | 0.01 |
| Methacryloxypropylsilane | 0.010 | 0.10 | 0.16 |

**Table 2. Polymerizable Dental Compositions of this Invention**

| **EXAMPLE** | **2D** | **2E** | **2F** |
|---|---|---|---|
| Heptane | | 10 | |
| Methyl Acetate | 90.7 | 77.2 | 78.0 |
| Compound of Example 1 | 3.6 | 2.0 | 2.4 |
| Cyclohexane dimethanol dimethacrylate | | 4.0 | |
| Cyclohexane dimethanol diacrylate | | | 3.0 |
| 1,6-Hexanediol dimethacrylate | | 2.0 | |
| 1,6-Hexanediol diacrylate | 1.62 | | 2.0 |
| Tetrahydrofurfuryl methacrylate | | | |
| 2,4,6- Trimethylbenzoyldiphenylphosphine oxide (LUCIRIN TPO) | 0.02 | 0.2 | |
| ELVACITE 2009 - Poly (MMA-co-EA) | 4.0 | 4.0 | 14.0 |
| Camphorquinone | 0.008 | 0.08 | 0.08 |
| N, N-dimethyl-aminoneopentyl acrylate | 0.027 | 0.27 | 0.27 |
| Methacrylic acid | 0.014 | 0.14 | 0.14 |
| Butylated hydroxytoluene | 0.001 | 0.01 | 0.01 |
| Methacryloxypropylsilane | 0.010 | 0.10 | 0.10 |

### Example 3

In the following Example 3, a baseplate for a denture was treated with the wax removal / surface treatment composition of this invention.

First, a plaster dental model (cast) of a patient's mouth was coated with a release agent and the cast was heated to 55°C in an oven. Eclipse® baseplate resin (available from Dentsply International Inc.) was applied and shaped onto the warm cast using finger pressure. Excess material was trimmed away to form a baseplate over the warm cast. The baseplate, as supported by the warm cast, was light-cured for 10 minutes in an Eclipse® processing unit (Dentsply International). The cast was soaked in water and then the cured baseplate was removed from the cast. After roughening-up the entire surface of the baseplate with a coarse bur, wax rims/bite blocks were placed on the baseplate and an initial try-in of the denture was performed. The bite blocks were used to set up the articulator and proper occlusion was registered. Then, a temporary wax set-up resin was applied to the baseplate, and the artificial teeth were pressed into the wax resin to form a denture. The denture was placed in flask and then was heated in water to "boil out" the wax. The remaining baseplate and teeth in the flask were rinsed several times with clean boiling water. Immediately after this rinsing step, the flask halves were placed on a bench top. After the flask stopped steaming (about 1 to 2 minutes), the baseplate was wiped to remove excess water.

While the flask was still hot, the above-described formulation of Example 2A (Table 1) was liberally brushed onto the baseplate using 3-4 applications.

After the final washing, the baseplate was placed in the bottom half-portion of the flask. The upper half-portion of the flask was closed over the bottom half-portion, and the flask remained closed in a ventilated area for 10 to 15 minutes. Then, the flask was opened and the baseplate was wiped to remove any excess water. After the flask was allowed to cool, it was trial packed with an Lucitone 199® acrylic denture base resin (available from Dentsply International Inc.). Then the flask, containing the baseplate in the bottom half portion, was closed. The flask was placed in a ventilated area for 10-15 minutes. The flask was opened and the baseplate was wiped to remove excess water. Then, Lucitone 199® acrylic liquid was applied to wet the surface of the baseplate seated in the flask. A final packing of the flask was performed using Lucitone 199® acrylic resin, and the flask was closed and allowed to bench set for 15 minutes. The denture was processed using a 2 hour short heat-cure method so that the Lucitone 199® acrylic resin could harden. As the upper and lower half-portions of the flask were pressed together, the Lucitone 199® acrylic resin, baseplate, and set of artificial teeth were bonded together. The flask was opened and the final denture with embedded artificial teeth was removed. A photograph of the final denture produced according to Example 3 is shown in FIG. 1A.

### Comparative Example A

In the following Comparative Example A, the same procedures for making a denture as described in above Example 3 were followed, except the baseplate was not treated with a wax removal / surface treatment composition of this invention. A photograph of the final denture produced according to Comparative Example A is shown in FIG. 1B.

As shown in Comparative Example A, there is some delamination of the layered material constituting the baseplate. It is believed that this delamination is due to the weak bonding effect between the baseplate surface and overlying acrylic composition. The weak bonding effect may be due to a contaminated baseplate surface. Particularly, it is believed that the interface may contain wax residue and other foreign debris, since the baseplate surface was not treated with the composition of this invention.

Workers skilled in the art will appreciate that various modifications can be made to the embodiments and description herein without departing from the scope of
the present invention. It is intended that all such modifications be covered by the appended claims.

## Claims

1. A method of making a dental appliance, comprising the steps of:
a) providing a baseplate having an outer surface;
b) molding a temporary wax layer over the outer surface of the baseplate and pressing a set of artificial teeth in the wax layer so as to form a try-in baseplate;
c) placing the try-in baseplate in a patient's mouth to evaluate the baseplate for fit and subsequently removing the baseplate therefrom;
d) treating the try-in baseplate with heat to substantially remove the temporary wax layer; **characterized by**
e) applying a first polymerizable acrylic composition to the baseplate and allowing the composition to at least partially diffuse into the baseplate, the first acrylic composition containing at least one solvent; and
f) applying a second polymerizable acrylic composition to the baseplate and pressing the baseplate, first and second acrylic compositions, and set of artificial teeth together and curing the acrylic resins so that they harden and form a dental appliance.

2. The method of claim 1, wherein the first acrylic composition comprises: a polymerizable acrylic compound; a polymerization initiation system capable of being activated by light or heat, for polymerizing the composition; and a solvent capable of at least partially diffusing into the surface of the baseplate.

3. The method of claim 2, wherein the polymerizable acrylic compound of the composition is a semi-crystalline material.

4. The method of claim 2, wherein the polymerization initiation system of the composition comprises a photoactive agent.

5. The method of claim 4, wherein the polymerization initiation system of the composition comprises camphorquinone.

6. The method of claim 4, wherein the polymerization initiation system of the composition comprises 2,4,6-trimethylbenzoyl-diphenyl-phosphine oxide.

7. The method of claim 2, wherein the polymerization initiation system of the composition comprises a heat-activated agent.

8. The method of claim 2, wherein the composition further comprises a filler material selected from the group of inorganic and organic materials and mixtures thereof.

9. The method of claim 2, wherein the solvent is selected from the group consisting of acetone, heptane, methyl acetate, methyl ethyl ketone, methyl propyl ketone, and tetrahydrofuran, and mixtures thereof.

10. The method of claim 1, wherein the try- in baseplate is placed in a dental flask and heated to substantially remove the temporary wax layer.

11. The method' of claim 1, wherein the first polymerizable acrylic composition is applied to the baseplate and set of artificial teeth.

12. The method of claim 1, wherein the dental appliance is a denture.

## Patentansprüche

1. Verfahren zur Herstellung einer Dentalvorrichtung, umfassend die folgenden Schritte:
a) Bereitstellen einer Grundplatte mit einer äußeren Oberfläche;
b) Ausformen einer temporären Wachsschicht über der äußeren Oberfläche der Grundplatte und Einpressen eines Sets künstlicher Zähne in die Wachsschicht, um eine Try-in-Grundplatte zu bilden:
c) Platzieren der Try-in-Grundplatte im Mund eines Patienten, um die Grundplatte nach ihrem Sitz zu beurteilen und nachfolgend Entfernen der Grundplatte aus dem Mund;
d) Behandeln der Try-in-Grundplatte mit Wärme, um die temporäre Wachsschicht im Wesentlichen zu entfernen; **gekennzeichnet durch**
e) Aufbringen einer ersten polymerisierbaren Acrylzusammensetzung auf die Grundplatte und Ermöglichen, dass die Zusammensetzung wenigstens teilweise in die Grundplatte diffundiert, wobei die erste Acrylzusammensetzung wenigstens ein Lösungsmittel enthält; und
f) Aufbringen einer zweiten polymerisierbaren Acrylzusammensetzung auf die Grundplatte und Zusammenpressen der Grundplatte, der ersten und zweiten Acrylzusammensetzungen und des Sets künstlicher Zähne und Härten der Acrylharze, so dass diese aushärten und eine Dentalvorrichtung bilden.

2. Verfahren nach Anspruch 1, wobei die erste Acrylzusammensetzung folgendes umfasst:
eine polymerisierbare Acrylverbindung; ein Polymerisationsinitiator-System, das durch Licht oder Wärme aktiviert werden kann, um die Zusammensetzung zu polymerisieren; und ein Lösungsmittel, das wenigstens teilweise in die Oberfläche der Grundplatte diffundieren kann.

3. Verfahren nach Anspruch 2, wobei die polymerisierbare Acrylverbindung der Zusammensetzung ein halbkristallines Material ist.

4. Verfahren nach Anspruch 2, wobei das Polymerisationsinitiator-System der Zusammensetzung ein photoaktives Mittel umfasst.

5. Verfahren nach Anspruch 4, wobei das Polymerisationsinitiator-System der Zusammensetzung Campherchinon umfasst.

6. Verfahren nach Anspruch 4, wobei das Polymerisationsinitiator-System der Zusammensetzung 2,4,6-Trimethylbenzoyl-diphenyl-phosphinoxid umfasst.

7. Verfahren nach Anspruch 2, wobei das Polymerisationsinitiator-System der Zusammensetzung ein durch Wärme aktivierbares Mittel umfasst.

8. Verfahren nach Anspruch 2, wobei die Zusammensetzung ferner ein Füllmaterial, ausgewählt aus der Gruppe von anorganischen und organischen Materialien und Mischungen derselben, umfasst.

9. Verfahren nach Anspruch 2, wobei das Lösungsmittel ausgewählt ist aus der Gruppe, bestehend aus Aceton, Heptan, Methylacetat, Methylethylketon, Methylpropylketon und Tetrahydrofuran und Mischungen derselben.

10. Verfahren nach Anspruch 1, wobei die Try-in-Grundplatte in einen Zahnprothesenbehälter gelegt und erwärmt wird, um die temporäre Wachsschicht im Wesentlichen zu entfernen.

11. Verfahren nach Anspruch 1, wobei die erste polymerisierbare Acrylzusammensetzung auf die Grundplatte und das Set der künstlichen Zähne aufgebracht wird.

12. Verfahren nach Anspruch 1, wobei die Dentalvorrichtung ein Gebiss ist.

## Revendications

1. Procédé de production d'un appareil dentaire, comprenant les étapes de :
a) fourniture d'une plaque de base ayant une surface extérieure ;
b) moulage d'une couche de cire temporaire sur la surface extérieure de la plaque de base et pression d'un ensemble de dents artificielles dans la couche de cire de manière à former une plaque de base d'essai ;
c) mise en place de la plaque de base d'essai dans la bouche d'un patient pour évaluer l'adaptation de la plaque de base et ensuite enlèvement de la plaque de base de la bouche de ce patient ;
d) traitement de la plaque de base d'essai par la chaleur pour éliminer substantiellement la couche de cire temporaire ; **caractérisé par**
e) l'application d'une première composition acrylique polymérisable à la plaque de base, en laissant ensuite la composition se diffuser au moins partiellement dans la plaque de base, la première composition acrylique contenant au moins un solvant ; et
f) l'application d'une seconde composition acrylique polymérisable à la plaque de base, et la pression de la plaque de base, des première et seconde compositions acryliques, et de l'ensemble de dents artificielles ensemble et le durcissement des résines acryliques de telle sorte qu'elles durcissent et forment un appareil dentaire.

2. Procédé suivant la revendication 1, dans lequel la première composition acrylique comprend : un composé acrylique polymérisable ; un système d'initiation de polymérisation pouvant être activé par la lumière ou la chaleur, pour polymériser la composition ; et un solvant capable de diffuser au moins partiellement dans la surface de la plaque de base.

3. Procédé suivant la revendication 2, dans lequel le composé acrylique polymérisable de la composition est une matière semi-cristalline.

4. Procédé suivant la revendication 2, dans lequel le système d'initiation de polymérisation de la composition comprend un agent photoactif.

5. Procédé suivant la revendication 4, dans lequel le système d'initiation de polymérisation de la composition comprend la camphoquinone.

6. Procédé suivant la revendication 4, dans lequel le système d'initiation de polymérisation de la composition comprend l'oxyde de 2,4,6-triméthylbenzoyl-diphényl-phosphine.

7. Procédé suivant la revendication 2, dans lequel le système d'initiation de polymérisation de la composition comprend un agent activé par la chaleur.

8. Procédé suivant la revendication 2, dans lequel la composition comprend en outre une matière de charge choisie dans le groupe consistant de matières inorganiques et organiques et de leurs mélanges.

9. Procédé suivant la revendication 2, dans lequel le solvant est choisi dans le groupe consistant en l'acétone, l'heptane, l'acétate de méthyle, la méthyléthylcétone, la méthylpropylcétone, le tétrahydrofuranne et leurs mélanges.

10. Procédé suivant la revendication 1, dans lequel la plaque de base d'essai est placée dans un récipient dentaire et chauffée pour éliminer substantiellement la couche de cire temporaire.

11. Procédé suivant la revendication 1, dans lequel la première composition acrylique polymérisable est appliquée à la plaque de base et à l'ensemble de dents artificielles.

12. Procédé suivant la revendication 1, dans lequel l'appareil dentaire est une prothèse dentaire.
